(19) Europäisches Patentamt
European Patent Office
Office européen des brevets

(11) Publication number: **0 130 829**
**B1**

# (12) EUROPEAN PATENT SPECIFICATION

(45) Date of publication of patent specification: **03.06.87**

(21) Application number: **84304498.3**

(22) Date of filing: **29.06.84**

(51) Int. Cl.[4]: **C 07 C 39/21,** C 07 C 43/215, C 07 C 33/00, C 07 C 15/40, C 07 C 49/225, C 07 C 69/21, C 07 C 69/618, C 07 C 25/24, C 07 D 309/12, C 07 D 317/50, C 07 H 15/20

## (54) Rooperol and its derivatives.

(30) Priority: **30.06.83 GB 8317850**

(43) Date of publication of application: **09.01.85 Bulletin 85/02**

(45) Publication of the grant of the patent: **03.06.87 Bulletin 87/23**

(84) Designated Contracting States: **AT BE CH DE FR GB IT LI LU NL SE**

(56) References cited:
**EP-A-0 092 226**
**AT-B- 340 884**
**GB-A-1 284 475**
**GB-A-2 120 650**
**US-A-4 320 236**

**TETRAHEDRON, vol. 38, no. 11, 1982, C. GALEFFI et al. "Research on African Medicinal Plants-II", pages 1683-1687**

**JOURNAL OF THE AMERICAN CHEMICAL SOCIETY, vol. 91, 1969, J. KLEIN et al. "Metalation reactions. I. Formation and charge distribution in conjugated propargylic dianions", pages 3094-3096**

(73) Proprietor: **ROOPEROL (NA) NV
Leidseplein 29 2nd Floor
NL-1017 PS Amsterdam (NL)**

(72) Inventor: **Drewes, Siegfried
76 Blackburrow Road
Pietermaritzburg Natal (ZA)**
Inventor: **Liebenberg, Roelof Wilke
16 Andre Street, Pierneef Park
Johannesburg, Transvaal (ZA)**

(74) Representative: **Oliver, Roy Edward et al
POLLAK MERCER & TENCH High Holborn House
52-54 High Holborn
London WC1V 6RY (GB)**

(56) References cited:
**BULLETIN DE LA SOCIETE CHIMIQUE DE FRANCE, vol. 43, 1928, GRIGNARD et al. "Recherches sur quelques carbures acétyléniques", pages 141-142**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 130 829 B1

**Description**

This invention relates to the chemical compound rooperol and to related compounds and their derivatives. Rooperol is an acetylenic compound and has the formula:

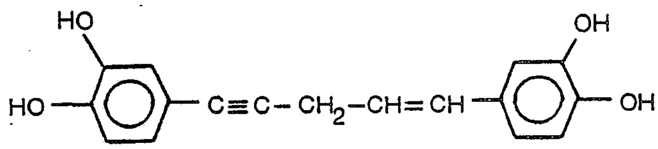

The compound, Hypoxoside, has the structure:

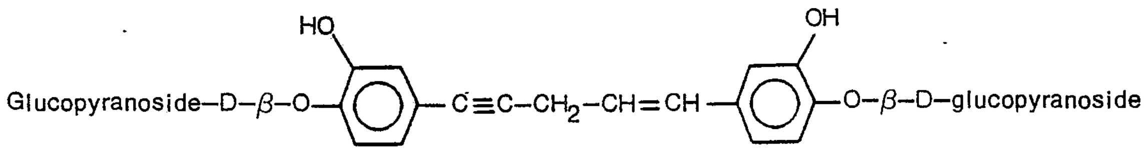

and may be named as a derivative of pentane as follows:
(E)-1,5-bis(3',4'-dihydroxyphenyl)pent-4-en-1-yne 4',4'-di-β-D-glycopyranoside. Hypoxoside is described in European Patent Application 83103765.0, published as EP—A2—0092226. In this patent application, the applicant reported that hypoxoside has anticancer activity. This compound was obtained by the extraction of *Hypoxis rooperi, H. Nitida, H. obtusa, H. rigidula, H. latifolia and other plants of the Hypoxidaceae species.* Hypoxoside is thus a di-β-D-glycopyranoside of rooperol.

Extracts of plants of the Hypoxis genus (Hypoxidaceae) have been found to be active in tests against Mouse P388 lymphocytic leukemia cell cultures (A. Barclay and R. C. Purdue: "Distribution of anticancer activity in higher plants". Cancer Treatment Reports, 1976, *60* 1081—1113 (Ref. I). In addition, extracts of *Hypoxis obtusa* have been reported to have been used by certain African people for combating urinary diseases. The isolation of hypoxoside from this plant has been reported, for instance, by G.B. Marini Bettolo, M. Patamia, M. Nicoletti, C. Galeffi and L. Messana in "Hypoxoside, a new glycoside of uncommon structure from *Hypoxis obtusa Busch*", Tetrahedron, 1982, *38*, 1683—1687 (Ref. II). This reference also describes the preparation of rooperol, under the alternative name of aglycone, by the hydrolysis of hypoxoside.

Acetylenic compounds occur widely in nature. Because of their antifungal, bacteriostatic and herbicidal properties as well as their potential application in medicine as hypnotic, sedative, anticonvulsant, analgesic, anti-inflammatory and hypotensive agents, considerable interest has been shown in the synthesis of these compounds. Numerous reviews cover the field of both natural and synthetic acetylenic compounds (the most comprehensive of these reviews are by K. E. Schulte and G. Rücker: "Synthetische und natürliche Acetylen-Verbindungen als Arzneistoffe," Fortschr. Arzneimettelforsch., (Progr. Drug Res,) 1970, *14*, 387—563 (Ref. III) and by O. G. Yashina and L. I. Vereshchagin: "Natural and Synthetic Acetylinic Antimycotics", Russian Chem. Revs. 1978, *47*, 307—317, (Trans. from Uspekhi Khimii, 1978, *47*, 557—575) (Ref. IV). Some acetylenic substances, although not structurally related to the compounds of the invention, have been found to be cytostatic and some have been used in the treatment of cancer (Ref. III). In addition, some natural and synthetic acetylenoids have the pent-4-en-1-yne central unit.

Although these particular compounds possess some of the above-mentioned biological properties, none of them has been reported to be cytostatic nor has any been described as potential or actual anticancer agents (Refs. III and IV). Compounds of this invention which have been previously synthesized are compound 3, by J. Klein and S. Brenner: J.A.C.S., 1969, *91*, 3094—3096 (Ref. V(a)) and by V. Grignard: Bull. Soc. Chim. Fr., 1928, *43*, 141—142, (Ref. V(b)), Compound 2 (Ref. V(a)) and Compounds 16 and 25 (Ref. IV), but a further reference (DE—PS 2025755 = GB—A—1284475 = US—A—3794689 = Chem. Abstr., 1971, *74*, 53260) (Ref. VI) describes different though closely related substances, of the formula:

$$(HC{\equiv}C)_2C(OH).Ar.$$

Similarly, closely-related 3-hydroxypent-4-en-1-ynes are disclosed in AT—PS 340884 and US—A—4320236. These compounds carry a terminal acetylenic hydrogen atom, which is replaced either by an aryl or an oxygen function, in the compounds of this invention.

It is an object of the present invention to prepare and describe the properties of compounds related to rooperol and, secondly, to describe the use of these compounds as anticancer agents. The anticancer properties of the compounds concerned have not been disclosed previously. Additionally, many of the compounds themselves have not been described before. The invention thus comprises anticancer compositions based upon the relevant compounds, those of the relevant compounds which have not been described previously and the use of the relevant compounds, whether known or new, in the preparation of anticancer compositions.

According to a first aspect of this invention, therefore, an anticancer composition is provided, comprising 10 to 500 mg of at least one active compound and inert formulation substances, characterised in that the active compound(s) is/are selected from those of the general formula:

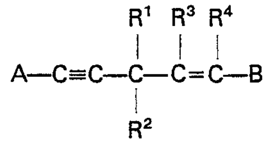

in which
(a) $R^1$ is a hydrogen atom or, taken together with $R^2$, is a keto group,
(b) $R^2$ is a hydrogen atom or a hydroxyl group,
(c) $R^3$ is a hydrogen atom or an acyl group,
(d) $R^4$ is a hydrogen or a halogen atom, and
(e) A and B are the same or different and A represents an alkyl or aryl group,
wherein all alkyl, aryl and acyl groups included in (c) and (e) may be variously substituted.

According to a second aspect of this invention, there is provided a group of compounds have the general formula:

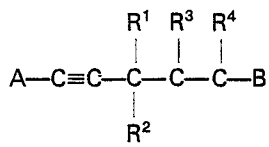

in which:
(a) $R^1$ is a hydrogen atom or, taken together with $R^2$, is a keto group,
(b) $R^2$ is a hydrogen atom or a hydroxyl group,
(c) $R^3$ is a hydrogen atom or an acyl group,
(d) $R^4$ is a hydrogen or a halogen atom, and
(e) A represents a phenyl, substituted phenyl or $CH_2R$ group (in which R is a hydrogen atom or an alkyl, aralkyl or acyl group), B represents a hydrogen atom or a phenyl, substituted phenyl, alkyl, furyl or $(CH_2)_nCOOH$ group (where n = 0—5) and, subject to these meanings, A and B are the same or different, subject to the provisos that:

(i) if $R^1 = R^2 = R^3 = R^4 = H$, then
(a) A and B are not both:

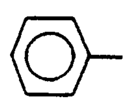 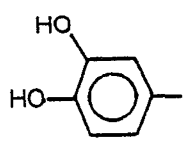 or its diacetate, 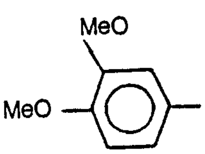 or 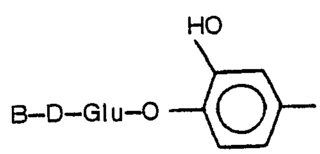

or its penta-acetate:
and
(b) A is not $C_6H_5$ when B is H or $CH_3$;

(ii) if $R^2 = OH$, $R^1 = R^3 = R^4 = H$ and $A = C_6H_5$, then B is not $CH_3$, $C_6H_5$ or m-chloro-phenyl; and

(iii) if $R^1$ and $R^2$ taken together are = O and $R^3 = R^4 = H$, then
(a) A and B both simultaneously are not phenyl, furyl, thienyl, pyridyl or norbornyl and
(b) A is not $C_6H_5$ and B is not H.

A number of examples of compounds according to the invention are listed in the following tables (1(a) and 1(b)). Rooperol per se is included as Product No. 1. Apart from Rooperol, all the compounds listed are among those which can be included in anticancer compositions according to the invention; the novel compounds listed are Products Nos. 4 to 7, 9, 10, 12, 15, 17, 18, 22, 23, 29 and 31.

TABLE 1(a)

| PRODUCT NO. | PRECURSORS AS ILLUSTRATED IN SCHEME X | Z | PRODUCT Y | FORMULA | mp/bp *nmr |
|---|---|---|---|---|---|
| 1 | | | 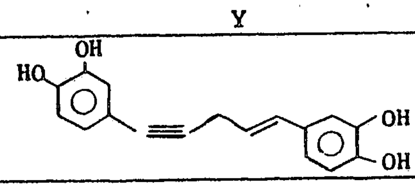 | $C_{17}H_{14}O_4$ | 148° |
| 2 | 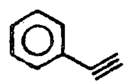 | 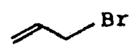 | 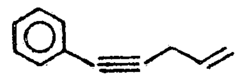 | $C_{11}H_{10}$ | 103°/20 Torr |
| 3 | 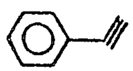 | 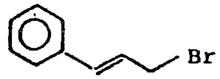 | 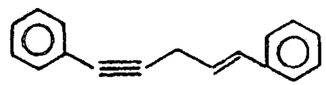 | $C_{17}H_{14}$ | 176°/2 Torr |
| 4 | 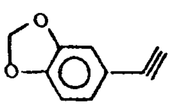 | 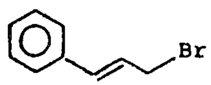 | 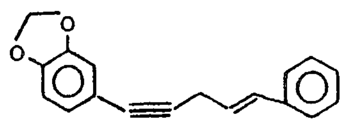 | $C_{18}H_{14}O_2$ | 49-50° |
| 5 | 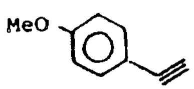 | 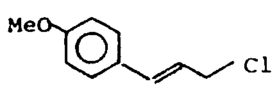 | 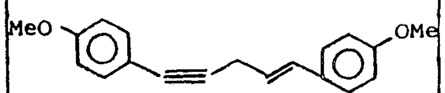 | $C_{19}H_{18}O_2$ | 45° |
| 6 | 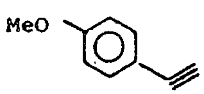 | 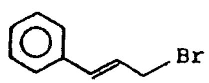 | 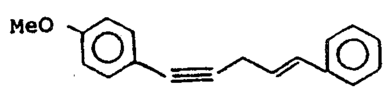 | $C_{18}H_{16}O$ | oil |

*nmr data per table 1(c)

TABLE 1(a) continued ...

| PRODUCT NO. | PRECURSORS AS ILLUSTRATED IN SCHEME X | Z | PRODUCT Y | FORMULA | mp/bp *nmr |
|---|---|---|---|---|---|
| 7 | 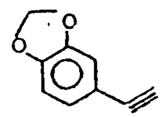 | 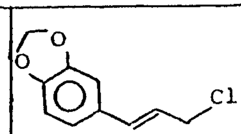 | 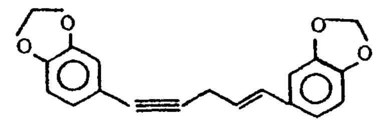 | $C_{19}H_{14}O_4$ | 59-60° |
| 8 | 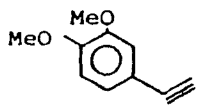 | 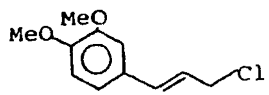 | 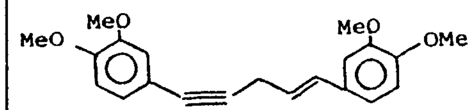 | $C_{21}H_{22}O_4$ | *nmr |
| 9 | 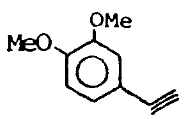 | 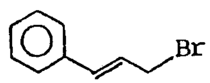 | 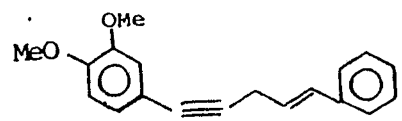 | $C_{19}H_{18}O_2$ | *nmr |
| 10 | 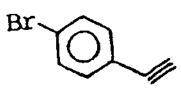 | 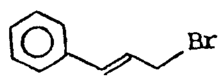 | 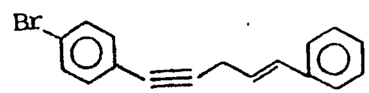 | $C_{17}H_{13}Br$ | 65-70° |
| 12 | 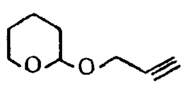 | 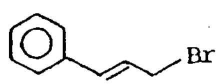 | 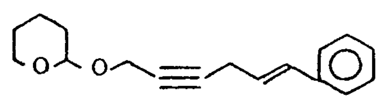 | $C_{17}H_{20}O_2$ | *nmr |

*nmr data per table 1(c)

TABLE 1(a) continued ...

| PRODUCT NO. | PRECURSORS AS ILLUSTRATED IN SCHEME X | Z | PRODUCT Y | FORMULA | mp/bp *nmr |
|---|---|---|---|---|---|
| 13 | 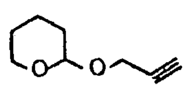 | 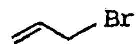 | 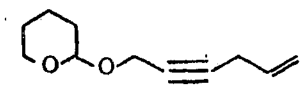 | $C_{11}H_{16}O_2$ | *nmr |
| 14 | 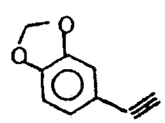 | 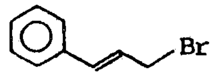 | 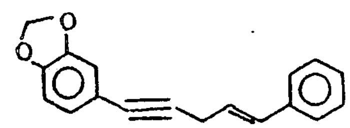 | $C_{18}H_{14}O_2$ | 49-50° |
| 15 | 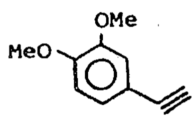 | 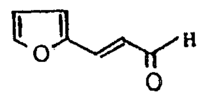 | 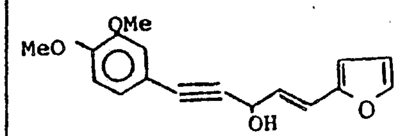 | $C_{17}H_{16}O_4$ | *nmr |
| 16 | 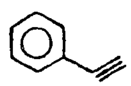 | 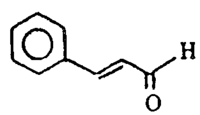 | 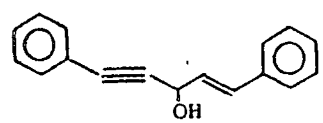 | $C_{17}H_{14}O$ | 69-71° |
| 17 | 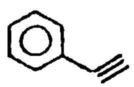 | 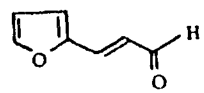 | 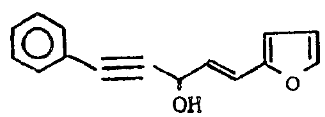 | $C_{15}H_{12}O_2$ | *nmr |
| 18 | 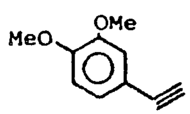 | 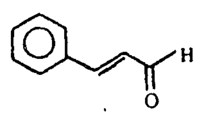 | 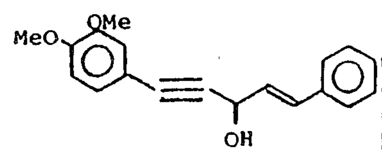 | $C_{19}H_{18}O_3$ | *nmr |

*nmr data per table 1(c)

TABLE 1(a) continued ...

| PRODUCT NO. | PRECURSORS AS ILLUSTRATED IN SCHEME X | Z | PRODUCT Y | FORMULA | mp/bp *nmr |
|---|---|---|---|---|---|
| 19 | 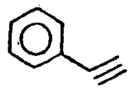 | 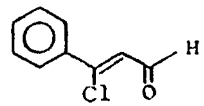 | 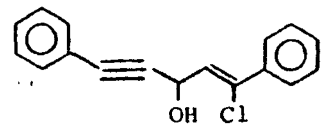 | $C_{17}H_{13}OCl$ | *nmr |
| 20 | 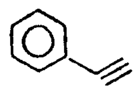 | 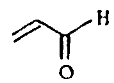 | 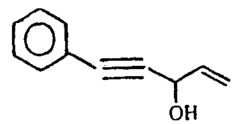 | $C_{11}H_{10}O$ | 98°/1.5 Torr |
| 22 | 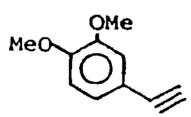 | 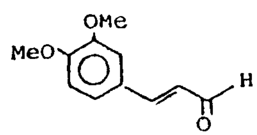 | 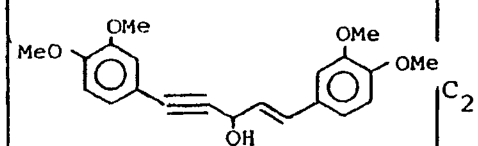 | $C_{21}H_{22}O_5$ | |
| 23 | 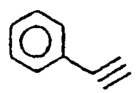 | 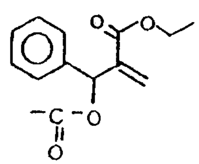 | 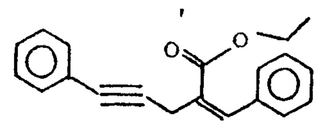 | $C_{20}H_{18}O_2$ | *nmr |

*nmr data per table 1(c)

DERIVATIVES OF PRODUCT NOS.1 to 23

*nmr data per Table 1 (c)

TABLE 1(b)

| PRODUCT NO. | PRODUCT | FORMULA | mp/bp *nmr |
|---|---|---|---|
| | | | |
| 25 | 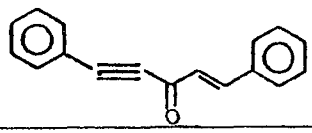 | $C_{17}H_{12}O$ | 69-71° |
| 26 | 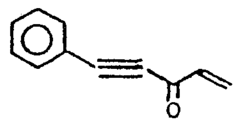 | $C_{11}H_8O$ | 105°/2 Torr |
| 27 | 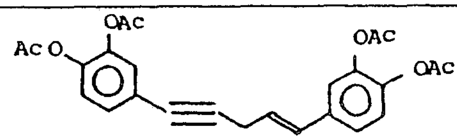 | $C_{25}H_{22}O_8$ | amorph |
| 28 | 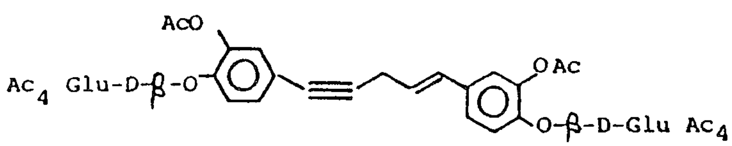 | $C_{49}H_{54}O_{24}$ | 134° |
| 29 | 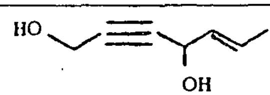 | $C_7H_{10}O_2$ | *nmr |
| 30 | 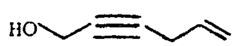 | $C_6H_8O$ | 60°/0.75 Torr |
| 31 | 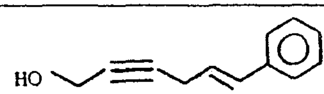 | $C_{12}H_{12}O$ | 127°/0.75 Torr |

TABLE 1 (c)

NMR DATA

Product 8

$\delta_H$ ($CDCl_3$; 80 MHz; TMS) 3.32 (2H, dd, $J_1$=5.4Hz $J_2$=<0.1Hz, $CH_2$—CH=CH), 3.85 (6H, s, 2 $OCH_3$) 3.88 (6H, s, 2 $OCH_3$), 5.99 and 6.19 (1H, dt, $J_1$=15Hz $J_2$=5.4Hz, $CH_2CH$=CH), 6.62 (1H, d, J=15Hz, CH=CH—ArH), 6.94 (6H, m, ArH).

Product 9

$\delta_H$ ($CDCl_3$; 60 MHz; TMS) 3.33 (2H, dd, $J_1$=5.0Hz $J_2$=<0.1Hz $CH_2$), 3.85 (6H; s, $OCH_3$), 6.06 and 6.32 (1H, dt, $J_1$=15.8Hz $J_2$=5.0Hz C—H—$CH_2$—), 6.55—7.43 (9H, m, CH—ArH).

Product 12

$\delta_H$ ($CCl_4$; 60 MHz; TMS) 1.23—1.80 (6H, m, THP, β, β', γ, $CH_2$) 3.08 (2H, dd, $J_1$=4Hz $J_2$=1.5Hz C≡C—$CH_2$—), 3.40—3.80 (2H, m, THP, α, $CH_2$), 4.27 (2H, t, J=2Hz O—$CH_2$), 4.73—4.90 (1H, m, THP, α, CH), 5.92 and 6.21 (1H, dt, $J_1$=15.8Hz $J_2$=5.4Hz, $CH_2CH$=C), 6.61 (1H, d, J=15.8Hz CH—ArH), 7.08—7.37 (5H, m, ArH).

Product 13

$\delta_H$ ($CCl_4$; 60 MHz; TMS) 1.55 (6H, m, THP, β, β', γ, $CH_2$) 2.9 (2H, m, C≡C—$CH_2$), 3.3—3.9 (2H, m, THP, α, $CH_2$), 4.1 (2H, m, $OCH_2C$≡C), 4.7 (1H, s, THP, α, CH), 5.05 and 5.3 (2H, d, J=9.1Hz, CH=$CH_2$), 5.45—6.0 (1H, m, CH=$CH_2$).

Product 15

$\delta_H$ ($CDCl_3$; 60 MHz; TMS) 2.65 (1H, s, OH), 3.8 (6H, s, 2 $OCH_3$), 5.2 (1H, d, J=5.7Hz, C≡C—CH), 6.05—7.3

(8H, m, CH=CH 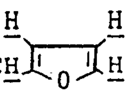 and ArH).

Product 17

$\delta_H$ ($CDCl_3$; 60 MHz; TMS) 2.65 (1H, s, OH), 5.2 (1H, d, J=5.9Hz, CH—OH), 6.0—6.7

),

(5H, m, CH=CH 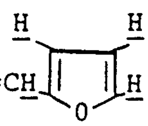 ),

7.0—7.6 (5H, m, ArH).

Product 18

$\delta_H$ ($CDCl_3$; 60 MHz; TMS) 3.8 (6H, s, 2 $OCH_3$), 5.2 (1H, d, C≡C—CH), 6.4 and 6.7 (2H, dd, $J_1$=6.9Hz $J_2$=<0.1Hz, CH=CH), 6.9 (1H, s, OH), 7.0—7.5 (8H, m, ArH).

Product 19

$\delta_H$ ($CDCl_3$; 60 MHz; TMS) 2.6 (1H, s, OH), 5.7 (1H, d, J=7.9Hz, C≡C—CH), 6.3 (1H, d, J=8.0Hz, CH=C), 7.1—7.7 (10H, m, ArH).

Product 23

$\delta_H$ ($CDCl_3$; 60 MHz; TMS), 1.36 (3H, t, J=7.5Hz, $CH_3$), 3.63 (2H; s, $CH_2$—C≡C), 4.33 (2H, d, J=7.5Hz $CH_2$—$CH_3$) 7.17—7.67 (10H, m, ArH) 7.83 (1H, s, C=CH).

Product 29

$\delta_H$ ($CDCl_3$; 60 MHz; TMS) 1.3 (3H, d, J=6.1Hz, $CH_3$), 3.1 (2H, s, 2OH), 4.15—4.5 (3H, m, CH=OH and C≡C—CH), 5.5 and 5.75 (1H, m, $CH_3CH$=CH), 6.0 and 6.3 (1H, dd, $J_1$=5.0Hz $J_2$=<0.1Hz, CH=CH—$CH_3$).

SCHEME

The compounds of the invention may be synthesised by the following scheme, in which A, B, $R^1$, $R^2$, $R^3$ and $R^4$ have the meanings described above; the carbon-carbon bonds in the cation Z are shown as dotted lines for simplicity:

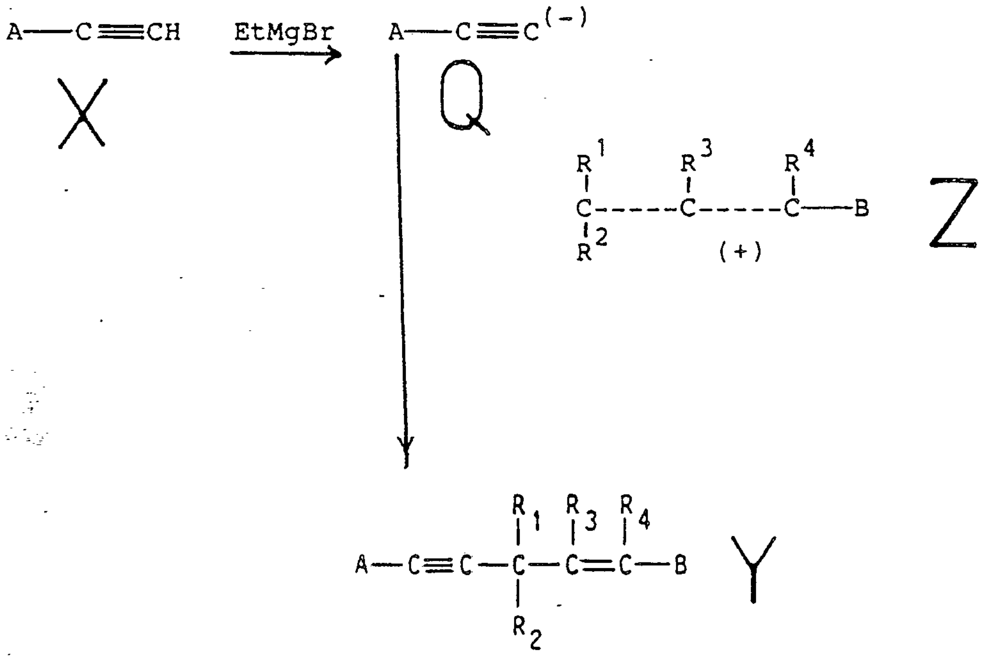


General Methods:

1. Bromoethane (1 mol equiv) is added dropwise to a stirred mixture of magnesium metal (1.05 mol equiv) and THF under a $N_2$ atmosphere. Once the exothermic reaction has subsided, the reaction mixture is refluxed for 10 minutes, cooled to 20°C and the alkyne X (as in Scheme) (1.05 mol equiv) added dropwise. Ethane is evolved. The mixture is then refluxed for 45 min., and cooled to 20°C. This provides anion Q in the reaction mixture as shown in the scheme.

2. *Products involving alkyl halide (Z) precursors:* Cuprous chloride (CUCl) is added to the Q containing mixture which is then stirred for 15 min. before the alkyl halide (Z) (1.05 mol equiv) is added dropwise. The green suspension is refluxed for 45 min. before aqueous ammonium chloride and KCN is added followed by extraction.

3. *Other products involving precursors Z.* The mixture which contains anion Q is cooled to 0°C and the compound Z (as in Scheme) is added dropwise maintaining the temperature between 0°C and 5°C during this procedure. Following addition of all the compound Z the mixture is stirred for 2½ hours at 25°C and then poured into a saturated aqueous solution of $NH_4Cl$ followed by extraction.

Preparation of Rooperol Product (1)

I. Hydrolysis of Hypoxoside (100 mg), in distilled water (6 ml, pH 6.3) with β-glucosidase (100 mg in 4 ml $H_2O$), at 37°C proved to be satisfactory. The 3′,3′,4′,4′-tetrahydroxyrooperol (1) had mp 148° (lit. mp 154—156°, ref. II).

II. Tetramethoxy product (8) (0.0075 mol), dry quinoline (0.25 mol) and TMSI (0.046 mol of ~92.5% solution) were added together under a nitrogen atmosphere and heated at 180°C for 70 min. After cooling and adition of 5% HCl the dark mixture was extracted with ether, the combined ether extracts were washed with 5% HCl (~550 ml). The washed ether extract was dried ($Na_2SO_4$), concentrated in vacuo, then heated at 40—50°C in methanol plus a few drops of water, until t.l.c. (benzene/acetone) (7:3) showed the high $R_F$ tetra (trimethyl silyl) ether of Product 1 to have disappeared with only Product 1 showing (c.f. Product 1 from plant material). The solvent was removed in vacuo at room temperature followed by purification of the residue on a medium pressure liquid chromatography column, eluting with benzene/acetone (7:3).

In Vitro Effects of Some Pent-4-en-1-ynes and Derivatives on Cell Cultures

The cell cultures were grown under standard conditions at 37°C in Eagle's Minimal Essential Medium (M.E.M., Gibco), with glutamine and non-essential amino acids, supplemented with 10% foetal calf serum (State Vaccine Institute, Cape Town). Cells for subcultures were obtained from 90—100% confluent cell cultures by trypsinisation with $Ca^{++}Mg^{++}$ — free EDTA-Dulbecco Buffer, containing 0.25% trypsin. Trypsin was inactivated by placing the harvested cell suspension into M.E.M. containing 10% foetal calf serum. The cell suspensions were then suitably diluted to the final volume, depending on the number of cells obtained

and the growth profile of the cell type. The diluted cell suspensions were then dispensed at the rate of 1.0 ml into prepared cell culture flasks containing 8.9 ml culture media. These cultures were incubated for 24 hours and the media changed before adding the test compounds.

The test compounds were dissolved in dimethylsulphoxide (Merck) and added at the rate of 0.1 ml to the media in a dilution to give the desired end concentration.

In each series of experiments, control cultures were treated with equivalent amounts of solvent without the test compound. The cultures were examined daily usually for a period of four days and cell growth and other cytopathic effects were records. Abnormal cell patterns and sizes were also determined during counting.

These included:

| | | |
|---|---|---|
| (i) | M (52) B | : Mouse Sarcoma |
| (ii) | Mel B16 BL06 | : Mouse Melanoma |
| (iii) | HOC | : Human Oesophagal Carcinoma |
| (iv) | HeLa ATCC No. CCL2 | : Human Cercival Cancer |
| (v) | P27 | : Human Mesothelioma Cells |
| (vi) | "Loots" cells | : Human Derived Adenocarcinoma |
| (vii) | Chang ATCC No. CCL20.2: | Chang Conjunctival cells |

The results are presented in tables 2 to 8.

Acute Toxicity Evaluation of Some Pent-4-en-1-ynes and Derivatives

Some compounds were subjected to preliminary acute toxicity tests in mice and rats. Solutions or suspensions of the selected compounds were prepared in vegetable oil. The volumes administered were kept constant at 10.0 ml per kg. In each case equal numbers of male and female animals were treated with a single dose. All animals were observed over 7 days for signs of toxic and/or pharmacodynamic effects. Necropsies were performed on all surviving animals. Results are presented in table 9.

TABLE 2

Inhibition of Cell Growth: Mouse Sarcoma M(52)B

| Product No. | Minimum Inhibitory Concentrations (Range) Which Produce Approximately 50% Cell Growth Reduction μg/ml Media |
|---|---|
| 1 | 12.5— 25.0 |
| 3 | 50.0— 75.0 |
| 7 | 60.0— 80.0 |
| 8 | 60.0— 80.0 |
| 16 | 20.0— 40.0 |
| 23 | 75.0—100.0 |

TABLE 3

Inhibition of Cell·Growth: Mouse Melanoma B16—BL6

| Product No. | Minimum Inhibitory Concentrations (Range) Which Produce Approximately 50% Cell Growth Reduction µg/ml Media |
|---|---|
| 1 | 10.0—25.0 |
| 16 | 20.0—50.0 |
| 25 | 12.5—25.0 |

TABLE 4

Inhibition of Cell Growth: Human Oesophagal Carcinoma HOC

| Product No. | Minimum Inhibitory Concentrations (Range) Which Produce Approximately 50% Cell Growth Reduction µg/ml Media |
|---|---|
| 1 | 25.0— 50.0 |
| 2 | 50.0—100.0 |
| 3 | 25.0— 50.0 |
| 5 | ≥25.0 |
| 6 | 10.0— 20.0 |
| 23 | 20.0— 50.0 |

TABLE 5

Inhibition of Cell Growth: Human Cercival Cancer HeLa ATCC No. CCL2

| Product No. | Minimurn Inhibitory Concentrations (Range) Which Produce Approximately 50% Cell Growth Reduction µg/ml Media |
|---|---|
| 1 | 20.0— 40.0 |
| 2 | 50.0— 75.0 |
| 3 | 40.0— 60.0 |
| 6 | 60.0—100.0 |
| 8 | 60.0—100.0 |
| 9 | 60.0—100.0 |
| 10 | 25.0— 50.0 |
| 13 | 50.0—100.0 |
| 15 | 25.0— 50.0 |
| 16 | 25.0— 50.0 |
| 17 | 20.0— 25.0 |
| 18 | 25.0— 50.0 |
| 19 | 20.0— 40.0 |
| 20 | 25.0— 50.0 |
| 23 | 60.0—100.0 |
| 25 | 5.0— 10.0 |
| 26 | 20.0— 40.0 |
| 29 | 50.0 |
| 30 | 50.0 |
| 31 | 25.0— 50.0 |

TABLE 6

Inhibition of Cell Growth: P27 — Human Mesothelioma

| Product No. | Minimum Inhibitory Concentrations (Range) Which Produce Approximately 50% Cell Growth Reduction µg/ml Media |
|---|---|
| 1 | 10.0—25.0 |
| 3 | 25.0—50.0 |
| 8 | 40.0—60.0 |
| 16 | 25.0—50.0 |
| 17 | 20.0—50.0 |

TABLE 7

Inhibition of Cell Growth: "Loot's" Cells Human Adenocarcinoma

| Product No. | Minimum Inhibitory Concentrations (Range) Which Produce Approximately 50% Cell Growth Reduction µg/ml Media |
|---|---|
| 1 | 12.5—25.0 |
| 16 | 12.5—25.0 |
| 23 | 25.0—50.0 |

TABLE 8

Inhibition of Cell Growth: Chang Conjunctival ATCC No. CCL20.2

| Product No. | Minimum Inhibitory Concentrations (Range) Which Produce Approximately 50% Cell Growth Reduction µg/ml Media |
|---|---|
| 1 | 20.0—25.0 |
| 23 | 25.0—75.0 |
| 25 | 2.5—10.0 |
| 26 | 2.5—10.0 |

TABLE 9

Acute Toxicity of Some Pent-4-en-1-ynes and Derivatives

| Product No. | Minimum Tolerated Single Dose mg/kg Body Weight | | |
|---|---|---|---|
| | Mice | Rats | Route |
| $P_2$ | ≥500 | ≥250 | P.O. |
| | ≥200 | ≥100 | I.P. |
| 2 | ≥1000 | 500 | P.O. |
| 3 | 500 | 1500 | P.O. |
| 16 | 500 | 500 | P.O. |
| 17 | 500 | 500 | P.O. |
| 19 | 250 | | P.O. |
| 23 | 750 | | P.O. |

NOTE:

≥ more than
P.O. by oral route
I.P. by intraperitoneal route

**Claims for the Contracting States: BE CH DE FR GB IT LI LU NL SE**

1. An anticancer composition, comprising 10 to 500 mg of at least one active compound and inert formulation substances, characterised in that the active compound(s) is/are selected from those of general formula:

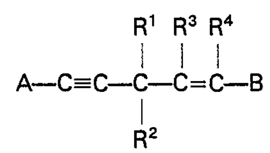

in which
(a) $R^1$ is a hydrogen atom or, taken together with $R^2$, is a keto group,
(b) $R^2$ is a hydrogen atom or a hydroxyl group,
(c) $R^3$ is a hydrogen atom or an acyl group,
(d) $R^4$ is a hydrogen or a halogen atom, and
(e) A and B are the same or different and A represents an alkyl or aryl group,
wherein all alkyl, aryl and acyl groups included in (c) and (e) may be variously substituted.

2. An anticancer composition according to claim 1, which comprises a pent-4-en-1-yne compound of the formula:

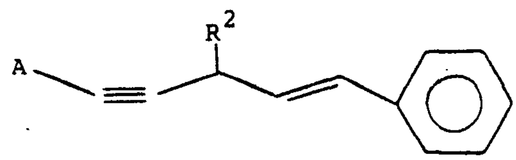

in which A is chosen from the group including a substituted phenyl group and a $CH_2OH$ group and $R^2$ is chosen from H and OH.

3. An anticancer composition according to claim 1, which comprises a pent-4-en-1-yne compound of the formula:

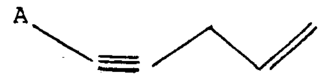

in which A is chosen from the group including a substituted phenyl group, a $CH_2$—OH group and H.

4. An anticancer composition according to claim 1, which comprises a pent-4-en-1-yne compound of the formula:

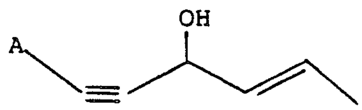

in which A is chosen from the group including a substituted phenyl group and a $CH_2$—OH group.

5. An anticancer composition according to claim 1, which comprises a pent-4-en-1-yne compound of the formula:

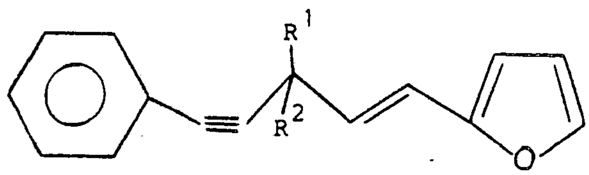

in which $R^1$ and $R^2$ are chosen from H and OH or, when taken together, are =0.

6. An anticancer composition according to claim 1, which comprises a pent-4-en-1-yne compound of the formula:

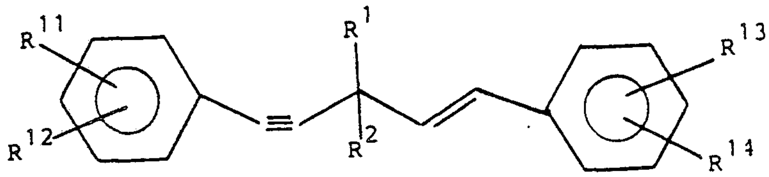

in which $R^1$ and $R^2$ are chosen from H and OH or, when taken together, are =0 and $R^{11}$, $R^{12}$, $R^{13}$ and $R^{14}$ are chosen from OMe, OH and O-glycoside.

7. An anticancer composition according to claim 1, which comprises a pent-4-en-1-yne compound of the formula:

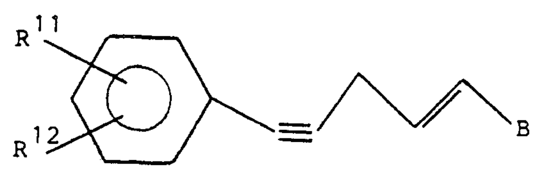

in which B is chosen from H, a phenyl group, a substituted phenyl group, an alkyl group or a $(CH_2)_nCOOH$ group, (where n = 0—5), and $R^{11}$ and $R^{12}$ are chosen from H, OH, OMe and O-glycoside.

8. An anticancer composition according to claim 1, which comprises a pent-4-en-1-yne compound of the formula:

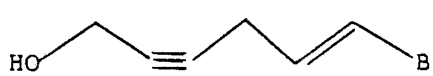

in which B is chosen from H, a phenyl group, a substituted phenyl group, an alkyl group and a $(CH_2)_nCOOH$ group (where n = 0—5).

9. An anticancer composition according to claim 1, which comprises a pent-4-en-1-yne compound of the formula:

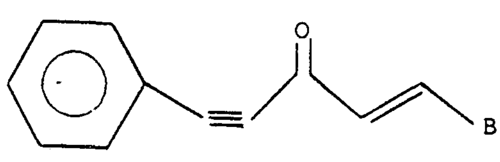

in which B is chosen from a substituted phenyl group, a $(CH_2)_nCOOH$ group, (where n = 0—5), and a furyl group.

10. An anticancer composition according to claim 1, which comprises a pent-4-en-1-yne compound of the formula:

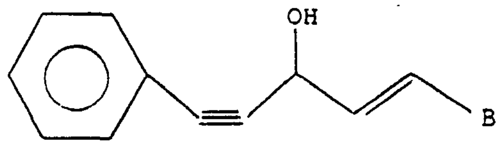

in which B is chosen from a substituted pheny group, an alkyl group, a $(CH_2)_nCOOH$ group, (where n = 0—5), and a furyl group.

11. An anticancer compound of the general formula:

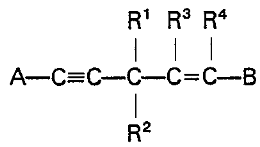

in which:

(a) $R^1$ is a hydrogen atom or, taken together with $R^2$, is a keto group,

(b) $R^2$ is a hydrogen atom or a hydroxyl group,

(c) $R^3$ is a hydrogen atom or an acyl group,

(d) $R^4$ is a hydrogen or a halogen atom, and

(e) A reprcesents a phenyl, substituted phenyl or $CH_2R$ group (in which R is a hydrogen atom or an alkyl, aralkyl or acyl group), B represents a hydrogen atom or a phenyl substituted phenyl, alkyl, furyl or $(CH_2)_nCOOH$ group (where n = 0—5) and, subject to these meanings, A and B are the same or different, subject to the provisos that:

(i) if $R^1 = R^2 = R^3 = R^4 = H$, then

(a) A and B are not both:

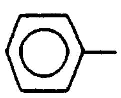 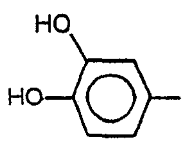 or its diacetate, 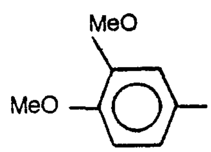 or 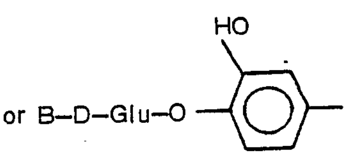

or its penta-acetate;

and

(b) A is not $C_6H_5$ when B is H or $CH_3$;

(ii) if $R^2 = OH$, $R^1 = R^3 = R^4 = H$ and $A = C_6H_5$, then B is not $CH_3$, $C_6H_5$ or m-chloro-phenyl; and

(iii) if $R^1$ and $R^2$ taken together are = 0 and $R^3 = R^4 = H$, then

(a) A and B both simultaneously are not phenyl, furyl, thienyl, pyridyl or norbornyl and

(b) A is not $C_6H_5$ and B is not H.

12. An anticancer compound comprising a pent-4-en-1-yne compound of the formula:

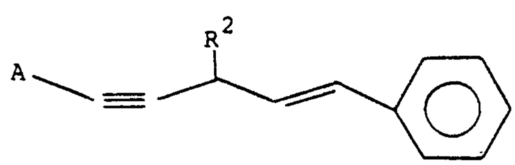

in which A is chosen from the group including a substituted phenyl group and a $CH_2OH$ group and $R^2$ is chosen from H and OH.

13. An anticancer compound comprising a pent-4-en-1-yne compound of the formula:

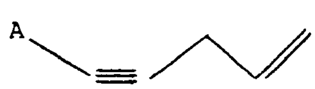

in which A is chosen from the group including a substituted phenyl group, a $CH_2$—OH group and H.

14. An anticancer compound comprising a pent-4-en-1-yne compound of the formula:

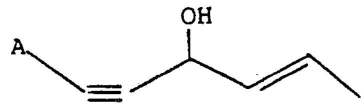

in which A is chosen from the group including a substituted phenyl group and a $CH_2$—OH group.

15. An anticancer compound comprising a pent-4-en-1-yne compound of the formula:

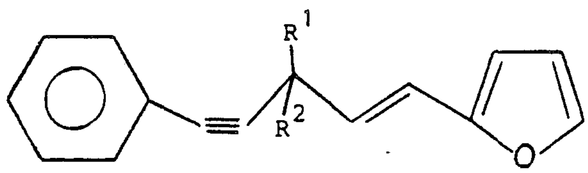

in which $R^1$ and $R^2$ are chosen from H and OH or, when taken together, are =0.

16. An anticancer compound comprising a pent-4-en-1-yne compound of the formula:

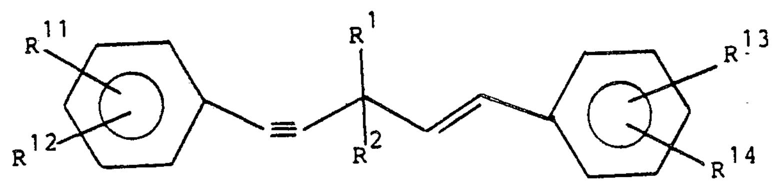

in which $R^1$ and $R^2$ are chosen from H and OH or, when taken together, are =0 and $R^{11}$, $R^{12}$, $R^{13}$ and $R^{14}$ are chosen from OMe, OH and O-glycoside.

17. An anticancer compound comprising a pent-4-en-1-yne compound of the formula:

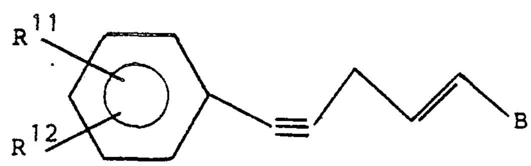

in which B is chosen from H, a phenyl group, a substituted phenyl group, an alkyl group or a $(CH_2)_nCOOH$ group, (where n = 0—5), and $R^{11}$ and $R^{12}$ are chosen from H, OH, OMe and O-glycoside.

18. An anticancer compound comprising a pent-4-en-1-yne compound of the formula:

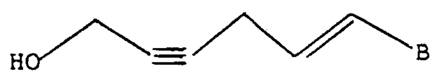

in which B is chosen from H, a phenyl group, a substituted phenyl group, an alkyl group and a $(CH_2)_nCOOH$ group, (where n = 0—5).

19. An anticancer compound comprising a pent-4-en-1-yne compound of the formula:

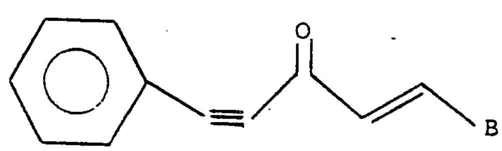

in which B is chosen from a substituted phenyl group, a $(CH_2)_nCOOH$ group (where n = 0—5) and a furyl group.

20. An anticancer compound comprising a pent-4-en-1-yne compound of the formula:

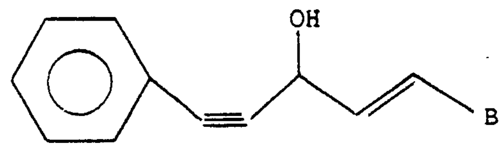

in which B is chosen from a substituted phenyl group, an alkyl group, a $(CH_2)_nCOOH$ group, (where $n = 0$—5), and a furyl group.

21. An anticancer compound, which comprises any one of the compounds identified as Products Nos. 4, 5, 6, 7, 9, 10, 12, 15, 17, 18, 22, 23, 29 and 31 as listed herein.

22. Use of at least one compound of the general formula defined in claim 1, in the preparation of an anticancer composition.

**Claims for the Contracting State: AT**

1. A method of preparation of an anticancer compound of the general formula:

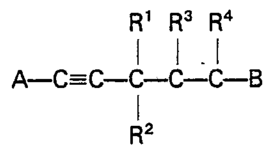

in which

(a) $R^1$ is a hydrogen atom or, taken together with $R^2$, is a keto group,

(b) $R^2$ is a hydrogen atom or a hydroxyl group,

(c) $R^3$ is a hydrogen atom or an acyl group,

(d) $R^4$ is a hydrogen or a halogen atom, and

(e) A and B are the same or different and A represents an alkyl or aryl group,

wherein all alkyl, aryl and acyl groups included in (c) and (e) may be variously substituted; characterised in that: a compound of the formula A—C≡CH is reacted with EtMgBr, to form the anion A—C≡$C^{(-)}$, and the anion is then reacted with a cation of the formula

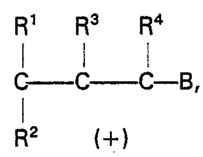

to give the final product, wherein A, B and $R^1$ to $R^4$ have the meanings defined above.

2. A method according to claim 1, in which the final product is a pent-4-en-1-yne compound of the formula:

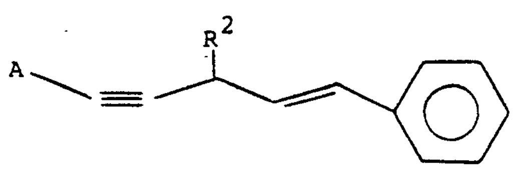

characterised in that A is chosen for the group including a substituted phenyl group and a $—CH_2OH$ group and $R^2$ is chosen from H and OH.

3. A method according to claim 1, in which the final product is a pent-4-en-1-yne compound of the formula:

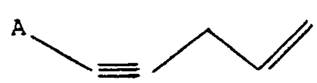

characterised in that A is chosen from the group including a substituted phenyl group, a $—CH_2OH$ group and H.

4. A method according to claim 1, in which the final product is a pent-4-en-1-yne compound of the formula:

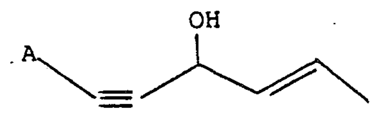

characterised in that A is chosen from the group including a substituted phenyl group and a $CH_2$—OH group.

5. A method according to claim 1, in which the final product is a pent-4-en-1-yne compound of the formula:

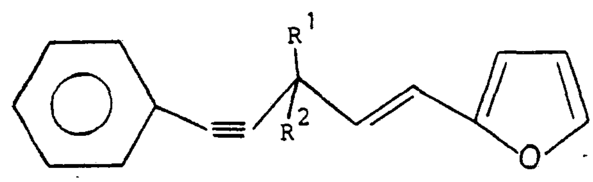

characterised in that $R^1$ and $R^2$ are chosen from H and OH or, when taken together, are =0.

6. A method according to claim 1, in which the final product is a pent-4-en-1-yne compound of the formula:

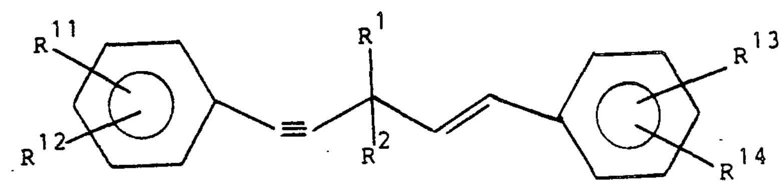

characterised in that $R^1$ and $R^2$ are chosen from H and OH, or when taken together, are =0 and $R^{11}$, $R^{12}$, $R^{13}$ and $R^{14}$ are chosen from OMe, OH and O-glycoside.

7. A method according to claim 1, in which the final product is a pent-4-en-1-yne compound of the formula:

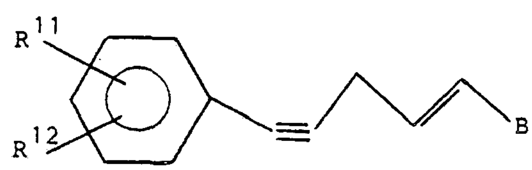

characterised in that B is chosen from H, a phenyl group, a substituted phenyl group, an alkyl group or a $(CH_2)_nCOOH$, (where n = 0—5), and $R^{11}$ and $R^{12}$ are chosen from H, OH, OMe and O-glycoside.

8. A method according to claim 1, in which the final product is a pent-4-en-1-yne compound of the formula:

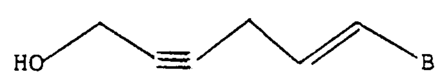

characterised in that B is chosen from H, a phenyl group, a substituted phenyl group, an alkyl group and a $(CH_2)_nCOOH$, (where n = 0—5).

9. A method according to claim 1, in which the final product is a pent-4-en-1-yne compound of the forumula:

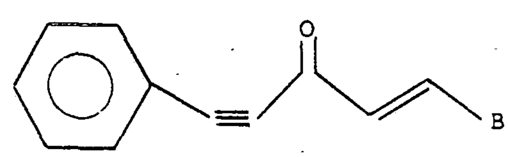

characterised in that B is chosen from a substituted phenyl group, a $(CH_2)_nCOOH$ (where n = 0—5) and a furyl group.

10. A method according to claim 1, in which the final product is a pent-4-en-1-yne compound of the formula:

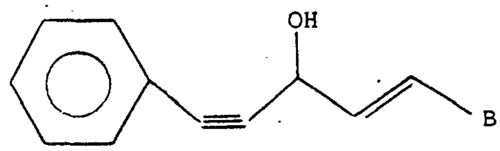

characterised in that B is chosen from a substituted phenyl group, an alkyl group, a $(CH_2)_nCOOH$ group, (where n = 0—5) and a furyl group.

**Patentansprüche für die Vertragsstaaten: BE CH DE FR GB IT LI LU NL SE**

1. Krebsbekämpfungsmittel, umfassend 10 bis 500 mg wenigstens einer aktiven Verbindung und inerte Formulierungssubstanzen, dadurch gekennzeichnet, daß die aktive(n) Verbindung(en) aus solchen der allgemeinen Formel:

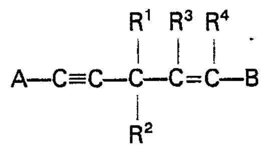

ausgewählt ist/sind, in der
(a) $R^1$ ein Wasserstoffatom oder, zusammensgefaßt mit $R^2$, eine Ketogruppe,
(b) $R^2$ ein Wasserstoffatom oder eine Hydroxylgruppe,
(c) $R^3$ ein Wasserstoffatom oder eine Acylgruppe,
(d) $R^4$ eine Wasserstoff- oder ein Halogenatom ist und
(e) A und B gleich oder verschieden sind und A eine Alkyl- oder Arylgruppe darstellt,
wobei sämtliche Alkyl-, Aryl- und Acylgruppen, die von (c) und (e) umfaßt werden, unterschiedlich substituiert sein können.

2. Krebsbekämpfungsmittel gemäß Anspruch 1, das eine Pent-4-en-1-yn-Verbindung der Formel:

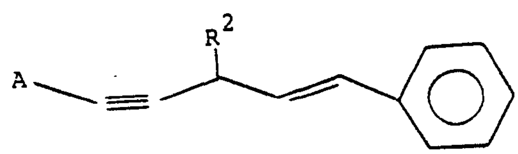

umfaßt, in der A ausgewählt ist aus der Gruppe umfassend eine substituierte Phenylgruppe und eine $CH_2OH$-Gruppe und $R^2$ ausgewählt ist aus H und OH.

3. Krebsbekämpfungsmittel gemäß Anspruch 1, das eine Pent-4-en-1-yn-Verbindung der Formel:

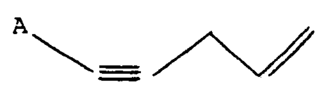

umfaßt, in der A ausgewählt ist aus der Gruppe umfassend eine substituierte Phenylgruppe, eine $CH_2$-OH-Gruppe und H.

4. Krebsbekämpfungsmittel gemäß Anspruch 1, das eine Pent-4-en-1-yn-Verbindung der Formel:

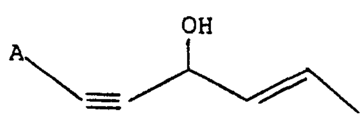

umfaßt, in der A ausgewählt ist aus der Gruppe umfassend eine substituierte Phenylgruppe und eine $CH_2$-OH-Gruppe.

5. Krebsbekämpfungsmittel gemäß Anspruch 1, das eine Pent-4-en-1-yn-Verbindung der Formel:

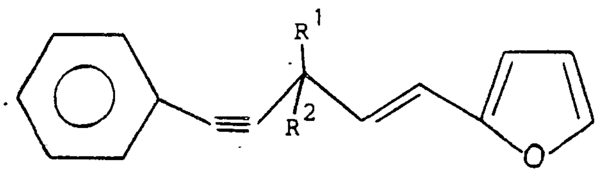

umfaßt, in der $R^1$ und $R^2$ ausgewählt sind aus H und OH oder zusammengenommen =0 sind.

6. Krebsbekämpfungsmittel gemäß Anspruch 1, das eine Pent-4-en-1-yn-Verbindung der Formel:

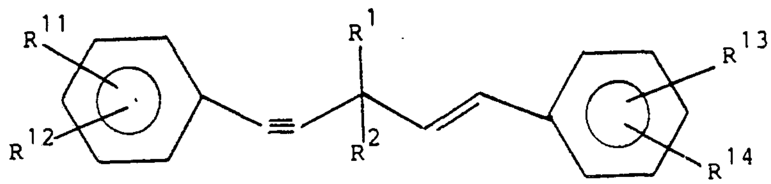

umfaßt, in der $R^1$ und $R^2$ ausgewählt sind aus H und OH oder zusammen genommen =0 sind und $R^{11}$, $R^{12}$, $R^{13}$ und $R^{14}$ ausgewählt sind aus OMe-, OH- und O-Glycosid.

7. Krebsbekämpfungsmittel gemäß Anspruch 1, das eine Pent-4-en-1-yn-Verbindung der Formel:

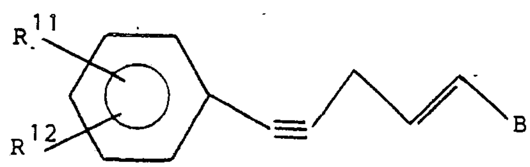

umfaßt, in der B ausgewählt is aus H, einer Phenylgruppe, einer substituierten Phenylgruppe, einer Alkylgruppe oder einer $(CH_2)_nCOOH$-Gruppe (wobei n = 0 bis 5 ist) und $R^{11}$ und $R^{12}$ ausgewählt ist aus H-, OH-, OMe- und O-Glycosid.

8. Krebsbekämpfungsmittel nach Anspruch 1, das eine Pent-4-en-1-yn-Verbindung der Formel:

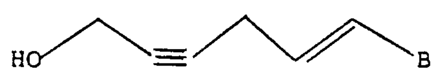

umfaßt, in der B ausgewählt ist aus H, einer Phenylgruppe, einer substituierten Phenylgruppe, einer Alkylgruppe und einer $(CH_2)_nCOOH$-Gruppe (wobei n = 0 bis 5 ist).

9. Krebsbekämpfungsmittel gemäß Anspruch 1, das eine Pent-4-en-1-yn-Verbindung der Formel:

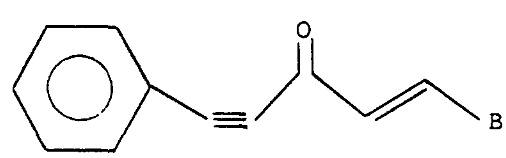

umfaßt, in der B ausgewählt ist aus einer substituierten Phenylgruppe, einer $(CH_2)_nCOOH$-Gruppe (wobei n = 0 bis 5 ist) und einer Furylgruppe.

10. Krebsbekämpfungsmittel gemäß Anspruch 1, das eine Pent-4-en-1-yn-Verbindung der Formel:

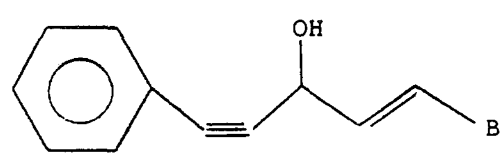

umfaßt, in der B ausgewählt ist aus einer substituierten Phenylgruppe, einer Alkylgruppe, einer $(CH_2)_nCOOH$-Gruppe (wobei = 0 bis 5 ist) und einer Furylgruppe.

11. Krebsbekämpfungsmittel der allgemeinen Formel:

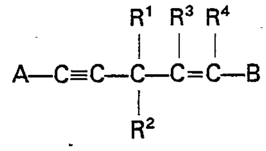

ausgewählt ist/sind, in der

(a) $R^1$ ein Wasserstoffatom oder, zusammengefaßt mit $R^2$, eine Ketogruppe,

(b) $R^2$ ein Wasserstoffatom oder eine Hydroxylgruppe,

(c) $R^3$ ein Wasserstoffatom oder eine Acylgruppe,

(d) $R^4$ eine Wasserstoff- oder Halogenatom ist und

(e) A eine Phenyl-, substituierte Phenyl- oder $CH_2R$-Gruppe (in der R ein Wasserstoffatom oder eine Alkyl-, Aralkyl- oder Acylgruppe ist) darstellt, B ein Wasserstoffatom oder eine Phenyl-, substituierte Phenyl-, Alkyl-, Furyl- oder $(CH_2)_nCOOH$-Gruppe (wobei n = 0 bis 5 ist) darstellt und — in Abhängigkeit von diesen Bedeutungen — A und B gleich oder verschieden sind, wobei abhängig von dem Vorbehalt, daß dann.

(i) wenn $R^1 = R^2 = R^3 = R^4 = H$ ist,

(a) A und B nicht beide

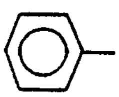 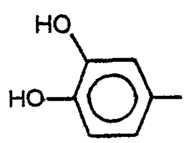 oder deren Diacetat, 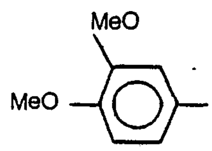 oder 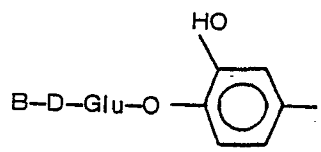

oder deren Pentaacetat ist und

(b) A nicht $C_6H_5$ ist, wenn B entweder H oder $CH_3$ ist;

(ii) wenn $R^2 = OH$, $R^1 = R^3 = R^4$ und $A = C_6H_5$ ist, B nicht $CH_3$, $C_6H_5$ oder m-Chlorphenyl ist; und

(iii) wenn $R^1$ und $R^2$ zusammengenommen =0 und $R^3 = R^4 = H$ sind,

(a) A und B beide gleichzeitig keine Phenyl-, Furyl-, Thienyl-, Pyridyl- oder Norbornylgruppe sind und

(b) A nicht $C_6H_5$ und B nicht H ist.

12. Krebsbekämpfungsmittel umfassend eine Pent-4-en-1-yn-Verbindung der Formel:

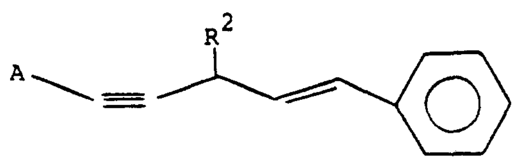

in der A ausgewählt ist aus der Gruppe umfassend eine substituierte Phenylgruppe und eine $CH_2OH$-Gruppe und $R^2$ ausgewählt ist aus H und OH.

13. Krebsbekämpfungsmittel umfassend eine Pent-4-en-1-yn-Verbindung der Formel:

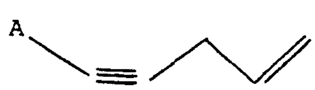

in der A ausgewählt ist aus der Gruppe umfassend eine substituierte Phenylgruppe, eine $CH_2$-OH-Gruppe und H.

14. Krebsbekämpfungsmittel umfassend eine Pent-4-en-1-yn-Verbindung der Formel:

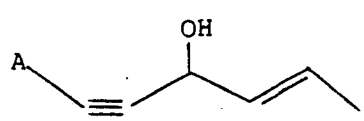

in der A ausgewählt ist aus der Gruppe umfassend eine substituierte Phenylgruppe und eine $CH_2$-OH-Gruppe.

15. Krebsbekämpfungsmittel umfassend eine Pent-4-en-1-yn-Verbindung der Formel:

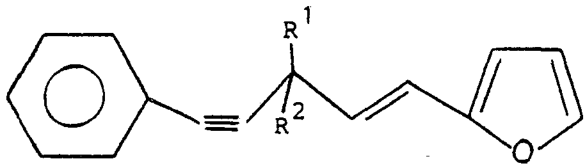

in der $R^1$ und $R^2$ ausgewählt sind aus H und OH oder zusammengenommen =0 sind.

16. Krebsbekämpfungsmittel umfassend eine Pent-4-en-1-yn-Verbindung der Formel:

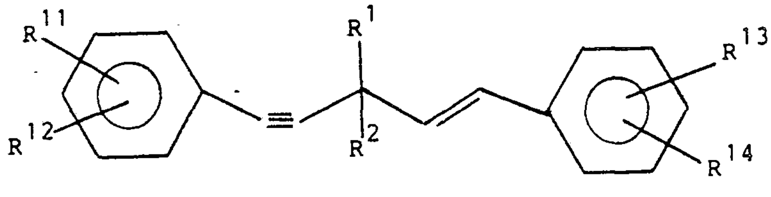

in der $R^1$ und $R^2$ ausgewählt sind aus H und OH oder zusammen genommen =0 sind und $R^{11}$, $R^{12}$, $R^{13}$ und $R^{14}$ ausgewählt sind aus OMe-, OH- und O-Glucosid.

17. Krebsbekämpfungsmittel umfassend eine Pent-4-en-1-yn-Verbindung der Formel:

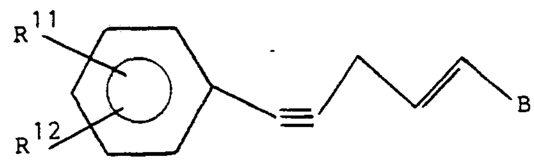

in der B ausgewählt ist aus H, einer Phenylgruppe, einer substituierten Phenylgruppe, einer Alkylgruppe oder einer $(CH_2)_nCOOH$-Gruppe (wobei n = 0 bis 5 ist) und $R^{11}$ und $R^{12}$ ausgewählt ist aus H-, OH-, OMe- und O-Glycosid.

18. Krebsbekämpfungsmittel umfassend eine Pent-4-en-1-yn-Verbindung der Formel:

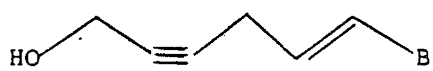

in der B ausgewählt is aus H, einer Phenylgruppe, einer substituierten Phenylgruppe, einer Alkylgruppe und einer $(CH_2)_nCOOH$-Gruppe (wobei n = 0 bis 5 ist).

19. Krebsbekämpfungsmittel umfassend eine Pent-4-en-1-yn-Verbindung der Formel:

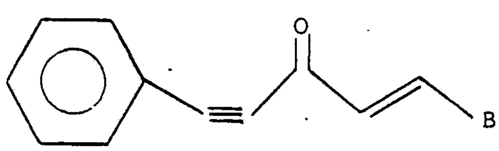

in der B ausgewählt ist aus einer substituierten Phenylgruppe, einer $(CH_2)_nCOOH$-Gruppe (wobei n = 0 bis 5 ist) und einer Furylgruppe.

20. Krebsbekämpfungsmittel umfassend eine Pent-4-en-1-yn-Verbindung der Formel:

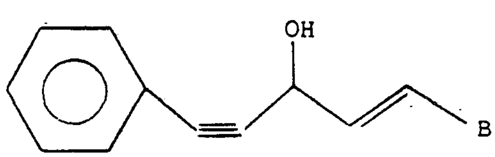

in der B ausgewählt ist aus einer substituierten Phenylgruppe, einer Alkylgruppe, einer $(CH_2)_nCOOH$-Gruppe (wobei n = 0 bis 5 ist) und einer Furylgruppe.

21. Krebsbekämpfungsmittel, das irgendeine der Verbindungen umfaßt, die als Produkt-Nr. 4, 5, 6, 7, 9, 10, 12, 15, 17, 18, 22, 23, 29 und 31, wie sie hierin aufgelistet sind, identifiziert sind.

22. Verwendung wenigstens einer Verbindung der allgemeinen in Anspruch 1 definierten Formel bei der Herstellung eines Krebsbekämpfungsmittels.

**Patentansprüche für den Vertragsstaat: AT**

1. Verfahren zur Herstellung eines Krebsbekämpfungsmittels der allgemeinen Formel:

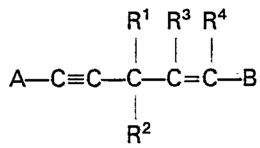

worin
(a) $R^1$ ein Wasserstoffatom oder, zusammensgefaßt mit $R^2$, eine Ketogruppe,
(b) $R^2$ ein Wasserstoffatom oder eine Hydroxylgruppe,
(c) $R^3$ ein Wasserstoffatom oder eine Acylgruppe,
(d) $R^4$ eine Wasserstoff- oder ein Halogenatom ist und
(e) A und B gleich oder verschieden sind A eine Alkyl- oder Arylgruppe darstellt,
wobei sämtliche Alkyl-, Aryl- und Acylgruppen, die von (c) und (e) umfaßt werden, unterschiedlich substituiert sein können; dadurch gekennzeichnet, daß: eine Verbindung der Formel A—C≡CH mit EtMgBr umgesetzt wird, um das Anion $A{-}C{\equiv}C^{(-)}$ zu bilden, und das Anion dann mit einem Kation der Formel

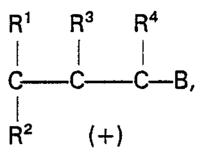

umgesetzt wird, um das Endprodukt zu ergeben, wobei, A, B und $R^1$ bis $R^4$ die oben definierten Bedeutungen haben.

2. Verfahren nach Anspruch 1, worin das Endprodukt eine Pent-4-en-1-yn-Verbindung der Formel

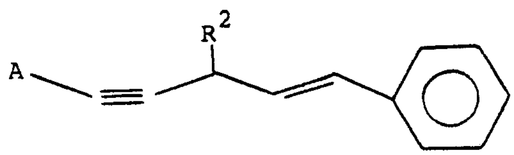

ist, dadurch gekennzeichnet, daß A ausgewählt ist aus der Gruppe umfassennd eine substituierte Phenylgruppe und eine —$CH_2OH$-Gruppe und $R^2$ ausgewählt ist aus H und OH.

3. Verfahren nach Anspruch 1, worin das Endprodukt eine Pent-4-en-1-yn-Verbindung der Formel

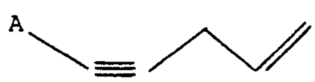

ist, dadurch gekennzeichnet, daß A ausgewählt ist aus der Gruppe, die eine substituierte Phenylgruppe, eine —$CH_2OH$-Gruppe und H umfaßt.

4. Verfahren nach Anspruch 1, worin das Endprodukt eine Pent-4-en-1-yn-Verbindung der Formel

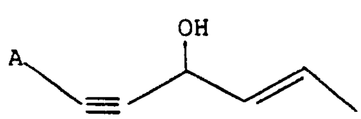

ist, dadurch gekennzeichnet, daß A ausgewählt ist aus der Gruppe, die eine substituierte Phenylgruppe und eine $CH_2$-OH-Gruppe umfaßt.

5. Verfahren nach Anspruch 1, worin das Endprodukt eine Pent-4-en-1-yn-Verbindung der Formel

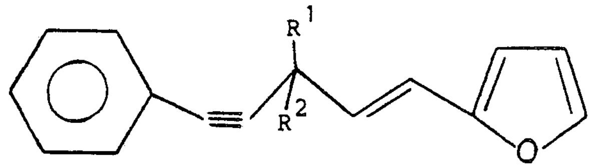

ist, dadurch gekennzeichent, daß $R^1$ und $R^2$ ausgewählt sind aus H und OH oder, wenn sie zusammengenommen werden, =0 bedeuten.

6. Verfahren nach Anspruch 1, worin das Endprodukt eine Pent-4-en-1-yn-Verbindung der Formel

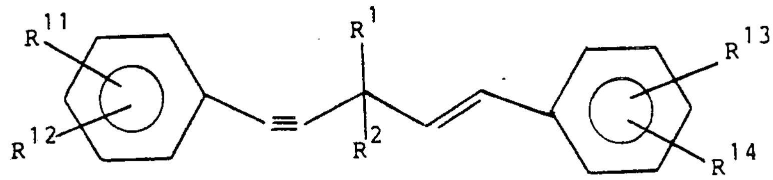

ist, dadurch gekennzeichnet, daß $R^1$ und $R^2$ ausgewählt sind aus H und OH, oder, wenn sie zusammengenommen werden, =0 bedeuten und $R^{11}$, $R^{12}$, $R^{13}$ und $R^{14}$ aus OMe, OH und O-Glycosid ausgewählt sind.

7. Verfahren nach Anspruch 1, worin das Endprodukt eine Pent-4-en-1-yn-Verbindung der Formel

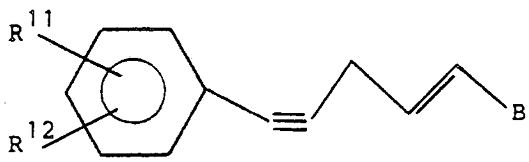

ist, dadurch gekennzeichnet, daß B ausgewählt ist aus H, einer Phenylgruppe, einer substituierten Phenylgruppe, einer Alkylgruppe oder einem $(CH_2)_nCOOH$, (worin n = 0—5) und $R^{11}$ und $R^{12}$ aus H, OH, OMe und O-Glycosid ausgewählt sind.

8. Verfahren nach Anspruch 1, worin das Endprodukt eine Pent-4-en-1-yn-Verbindung der Formel

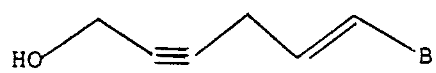

ist, dadurch gekennzeichnet, daß B ausgewählt ist aus H, einer Phenylgruppe, einer substituierten Phenylgruppe, einer Alkylgruppe und einem $(CH_2)_nCOOH$, (worin n = 0—5).

9. Verfahren nach Anspruch 1, worin das Endprodukt eine Pent-4-en-1-yn-Verbindung der Formel

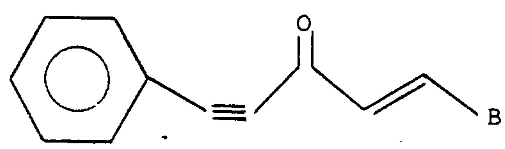

ist, dadurch gekennzeichnet, daß B ausgewählt ist aus einer substituierten Phenylgruppe, einem $(CH_2)_nCOOH$ (worin n = 0—5) und einer Furylgruppe.

10. Verfahren nach Anspruch 1, worin das Endprodukt eine Pent-4-en-1-yn-Verbindung der Formel:

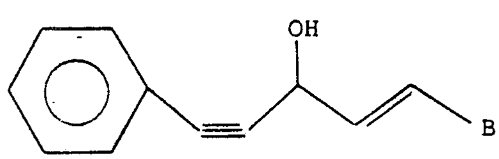

ist, dadurch gekennzeichnet, daß B ausgewählt ist aus einer substituierten Phenylgruppe, einer Alkylgruppe, einer $(CH_2)_nCOOH$-Gruppe (worin n = 0—5) und einer Furylgruppe.

**Revendications pour les Etats contractants: BE CH DE FR GB IT LI LU NL SE**

1. Une composition anticancéreuse comprenant 10 à 500 mg d'au moins un composé actif et des substances inertes de présentation, caractérisée en ce que le(s) composé(s) actif(s) est(sont) choisi(s) parmi ceux de formule générale:

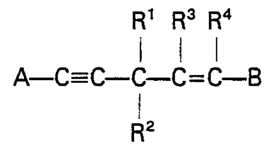

dans laquelle

(a) $R^1$ est un atome d'hydrogène ou représente avec $R^2$ un groupe céto,

(b) $R^2$ est un atome d'hydrogène ou un groupe hydroxyle,

(c) $R^3$ est un atome d'hydrogène ou un groupe acyle,

(d) $R^4$ est un atome d'hydrogène ou d'halogène, et

(e) A et B sont semblables ou différents et A représente un groupe alcoyle ou aryle,

où tous les groupes alcoyles, aryles et acyles compris dans (c) et (e) peuvent être diversement substitués.

2. Une composition anticancéreuse selon la revendication 1 qui comprend un composé pent-4-ène-1-yne de formule:

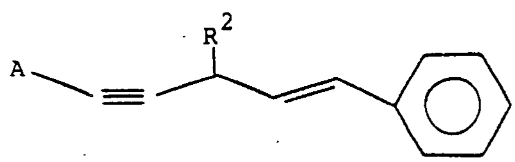

dans laquelle A est choisi dans le groupe comprenant un groupe phényle substitué et un groupe $CH_2OH$ et $R^2$ est choisi parmi H et OH.

3. Une composition anticancéreuse selon la revendication 1 qui comprend un composé pent-4-ène-1-yne de formule:

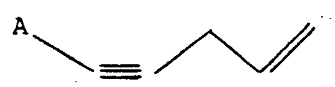

dans laquelle A est choisi dans le groupe comprenant un groupe phényle substitué, un groupe $CH_2$—OH et H.

4. Une composition anticancéreuse selon la revendication 1 qui comprend un composé pent-4-ène-1-yne de formule:

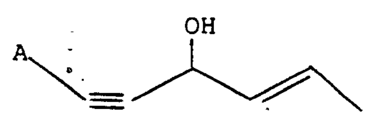

dans laquelle A est choisi dans le groupe comprenant un groupe phényle substitué et un groupe $CH_2$—OH.

5. Une composition anticancéreuse selon la revendication 1 qui comprend un composé pent-4-ène-1-yne de formule:

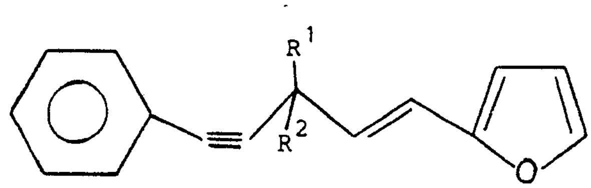

dans le laquel $R^1$ et $R^2$ sont choisis parmi H et OH ou, pris ensemble, sont =O.

6. Une composition anticancéreuse selon la revendication 1 qui comprend un composé pent-4-ène-1-yne de formule:

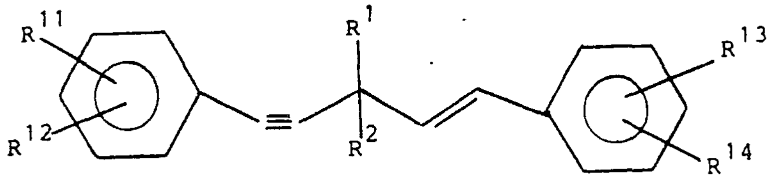

dans laquelle $R^1$ et $R^2$ sont choisis parmi H et OH ou, pris ensemble, sont =O et $R^{11}$, $R^{12}$, $R^{13}$ et $R^{14}$ sont choisis parmi OMe, OH et O-glycoside.

7. Une composition anticancéreuse selon la revendication 1 qui comprend un composé pent-4-ène-1-yne de formule:

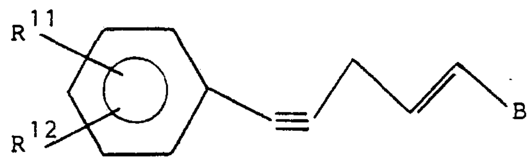

dans laquelle B est choisi parmi H, un groupe phényle, un groupe phényle substitué, un groupe alcoyle ou un groupe $(CH_2)_nCOOH$ (où n = 0—5), et $R^{11}$ et $R^{12}$ sont choisis parmi H, OH, OMe et O-glycoside.

8. Une composition anticancéreuse selon la revendication 1 qui comprend un composé pent-4-ène-1-yne de formule:

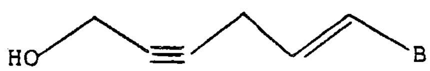

dans laquelle B est choisi parmi H, un groupe phényle, un groupe phényle substitué, un groupe alcoyle et un groupe $(CH_2)_nCOOH$ (où n = 0—5).

9. Une composition anticancéreuse selon la revendication 1 qui comprend un composé pent-4-ène-1-yne de formule:

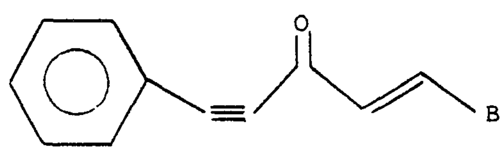

dans laquelle B est choisi parmi un groupe phényle substitué, un groupe alcoyle ou un groupe $(CH_2)_nCOOH$ (où n = 0—5) et un groupe furyle.

10. Une composition anticancéreuse selon la revendication 1 qui comprend un composé pent-4-ène-1-yne de formule:

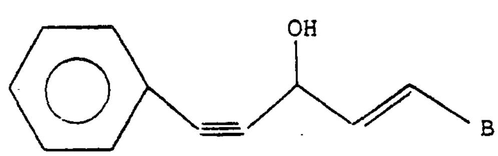

dans laquelle B est choisi parmi un groupe phényle substitué, un groupe alcoyle ou un groupe $(CH_2)_nCOOH$ (où n = 0—5) et un groupe furyle.

11. Un composé anticancéreaux de formule générale:

$$\begin{array}{c} \quad\quad\quad\;\; R^1\;\; R^3\;\; R^4 \\ \quad\quad\quad\;\; |\quad\; |\quad\; | \\ A—C{\equiv}C—C—C{=}C—B \\ | \\ R^2 \end{array}$$

dans laquelle:

(a) $R^1$ est un atome d'hydrogène ou forme avec $R^2$ un groupe céto,

(b) $R^2$ est un atome d'hydrogène ou un groupe hydroxyle,

(c) $R^3$ est un atome d'hydrogène ou un groupe acyle,

(d) $R^4$ est un atome d'hydrogène ou d'halogène, et

(e) A représente un groupe phényle, phényle substitué ou $CH_2R$ (où R est un atome d'hydrogène ou un groupe alcoyle, aralcoyle ou acyle), B représente un atome d'hydrogène ou un groupe phényle, phényle substitué, alcoyle, furyle ou $(CH_2)_nCOOH$ (où n = 0—5) et, en accord avec ces significations, A et B sont semblables ou différents,

sous réserve que:

(i) si $R^1 = R^2 = R^3 = R^4 = H$, alors

(a) A et B ne sont pas tous deux:

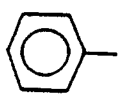 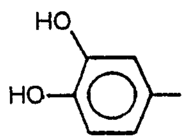 ou son diacétate 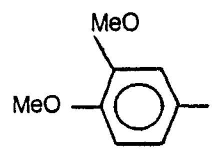 ou 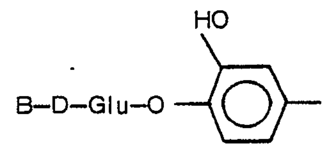

ou son pentaacétate; et

(b) A n'est pas $C_6H_5$ lorsque B est H ou $CH_3$;

(ii) si $R^2 = OH$, $R^1 = R^3 = R^4 = H$ et $A = C_6H_5$, alors B n'est pas $CH_3$, $C_6H_5$ ou m-chlorophényle; et

(iii) si $R^1$ et $R^2$ pris ensemble sont =O et $R^3 = R^4 = H$, alors

(a) A et B ne sont pas tous deux simultanément un phényle, un furyle, un thiényle, un pyridyle ou un norbornyle et

(b) A n'est pas $C_6H_5$ et B n'est pas H.

12. Un composé anticancéreux comprenant un composé pent-4-ène-1-yne de formule:

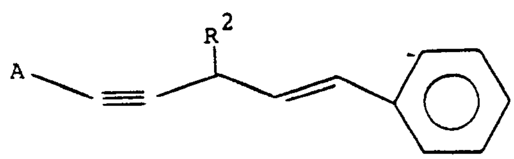

dans laquelle A est choisi dans le groupe comprenant un groupe phényle substitué et un groupe $CH_2OH$ et $R^2$ est choisi parmi H et OH.

13. Un composé anticancéreux comprenant un composé pent-4-ène-1-yne de formule:

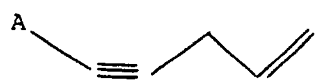

dans laquelle A est choisi dans le groupe comprenant un groupe phényle substitué, un groupe $CH_2$—OH et H.

14. Un composé anticancéreux comprenant un composé pent-4-ène-1-yne de formule:

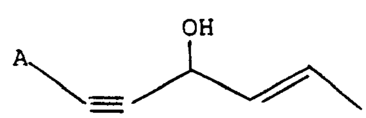

dans laquelle A est choisi dans le groupe comprenant un groupe phényle substitué et un groupe $CH_2$—OH.

15. Un composé anticancéreux comprenant un composé pent-4-ène-1-yne de formule:

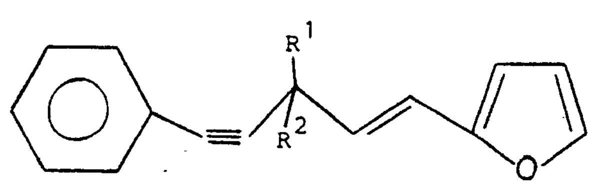

dans laquelle $R^1$ et $R^2$ sont choisis parmi H et OH ou, pris ensemble, sont =O.

16. Un composé anticancéreux comprenant un composé pent-4-ène-1-yne de formule:

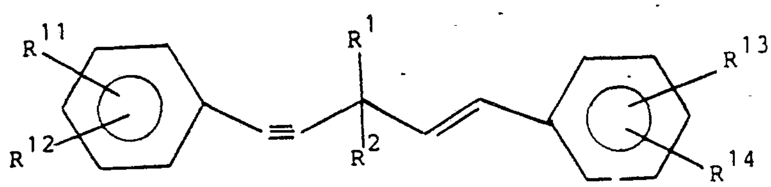

dans laquelle $R^1$ et $R^2$ sont choisis parmi H et OH ou, pris ensemble, sont =O et $R^{11}$, $R^{12}$, $R^{13}$ et $R^{14}$ sont choisis parmi OMe, OH et O-glycoside.

17. Un composé anticancéreux comprenant un composé pent-4-ène-1-yne de formule:

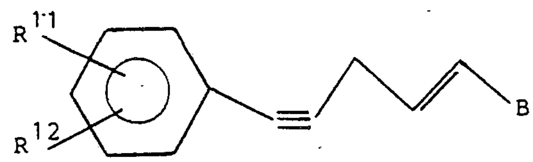

dans laquelle B est choisi parmi H, un groupe phényle, un groupe phényle substitué, un groupe alcoyle ou un groupe $(CH_2)_nCOOH$ (où n = 0—5) et $R^{11}$ et $R^{12}$ sont choisis parmi H, OH, OMe et O-glycoside.

18. Un composé anticancéreux comprenant un composé pent-4-ène-1-yne de formule:

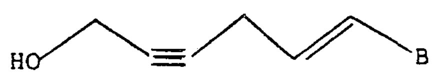

dans laquelle B est choisi parmi H, un groupe phényle, un groupe phényle substitué, un groupe alcoyle et un groupe $(CH_2)_nCOOH$ (où n = 0—5).

19. Un composé anticancéreux comprenant un composé pent-4-ène-1-yne de formule:

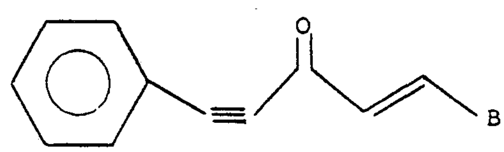

dans laquelle B est choisi parmi un groupe phényle substitué, $(CH_2)_nCOOH$ (où n = 0—5) et un groupe furyle.

20. Un composé anticancéreux comprenant un composé pent-4-ène-1-yne de formule:

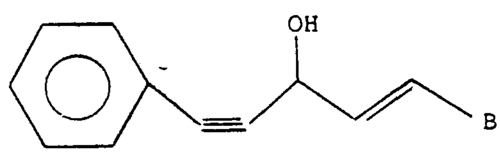

dans laquelle B est choisi parmi un groupe phényle substitué, un groupe alcoyle, un groupe $(CH_2)_nCOOH$ (où n = 0—5) et un groupe furyle.

21. Un composé anticancéreux qui comprend l'un quelconque des composés identifiés comme produits No. 4, 5, 6, 7, 9, 10, 12, 15, 17, 18, 22, 23, 29 et 31 comme énuméré ici.

22. Emploi d'au moins un composé répondant à la formule générale définie dans la revendication 1 dans la préparation d'une composition anticancéreuse.

**Revendications pour l'Etat contractant: AT**

1. Procédé de préparation d'une composition anticancéreuse de formule générale:

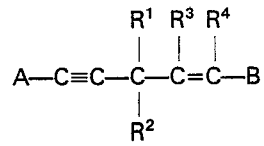

dans laquelle
(a) $R^1$ est un atome d'hydrogène ou représente avec $R^2$ un groupe céto,
(b) $R^2$ est un atome d'hydrogène ou un groupe hydroxyle,
(c) $R^3$ est un atome d'hydrogène ou un groupe acyle,
(d) $R^4$ est un atome d'hydrogène ou d'halogène, et
(e) A et B sont semblables ou différents et A représente un groupe alcoyle ou aryle,
où tous les groupes alcoyles, aryles et acyles compris dans (c) et (e) peuvent être diversement substitués; caractérisé en ce que:

un composé de formule $A—C\equiv CH$ est mis en réaction avec l'EtMgBr, pour former l'anion $A—C\equiv C^{(-)}$ et l'anion est alors mis en réaction avec un cation de formule

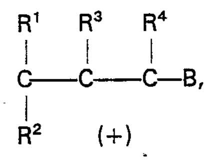

pour donner le produit final, dans lequel A, B et $R^1$ à $R^4$ ont les significations définies ci-dessus.

2. Procédé selon la revendication 1, dans lequel le produit final est un composé pent-4-ène-1-yne de formule

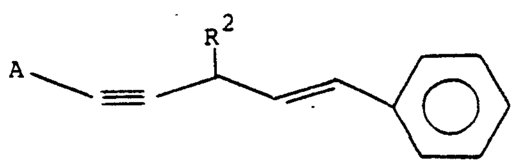

caractérisé en ce que A est choisi dans le groupe comprenant un groupe phényle substitué et un groupe $—CH_2OH$ et $R^2$ est choisi parmi H et OH.

3. Procédé selon la revendication 1, dans lequel le produit final est un composé pent-4-ène-1-yne de formule

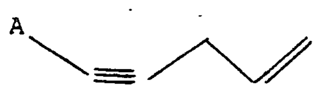

caractérisé en ce que A est choisi dans le groupe comprenant un groupe phényle substitué et un groupe $—CH_2OH$ et H.

4. Procédé selon la revendication 1, dans lequel le produit final est un composé pent-4-ène-1-yne de formule:

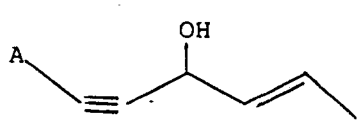

caractérisé en ce que A est choisi parmi le groupe comprenant un groupe phényle substitué et un groupe $CH_2—OH$.

5. Procédé selon la revendication 1, dans lequel le produit final est un composé pent-4-ène-1-yne de formule:

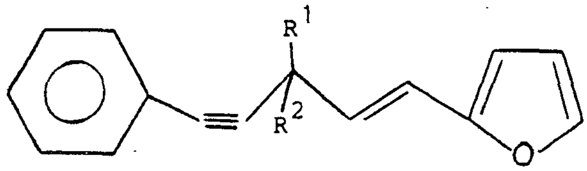

caractérisé en ce que $R^1$ et $R^2$ sont choisis parmi H et OH ou pris ensemble, sont = O.

6. Procédé selon la revendication 1, dans lequel le produit final est un composé pent-4-ène-1-yne de formule:

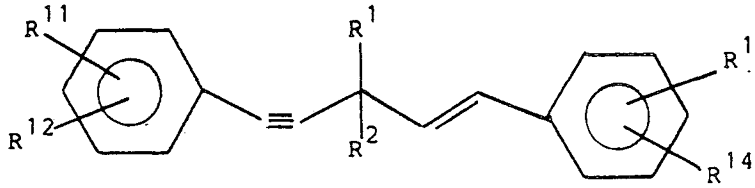

caractérisé en ce que $R^1$ et $R^2$ sont choisis parmi H et OH ou, pris ensemble, sont = O et $R^{11}$, $R^{12}$, $R^{13}$ et $R^{14}$ sont choisis parmis OMe, OH et O-glycoside.

7. Procédé selon la revendication 1, dans lequel le produit final est un composé pent-4-ène-1-yne de formule:

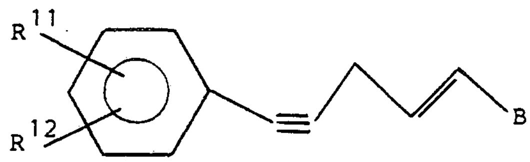

caractérisé en ce B est choisis parmi H, un groupe phényle, un groupe phényle substitué, un groupe alcoyle ou un groupe $(CH_2)_nCOOH$ (où n = 0—5), et $R^{11}$ et $R^{12}$ sont chosis parmi H, OH, OMe ou O-glycoside.

8. Procédé selon la revendication 1, dans lequel le produit final est un composé pent-4-ène-1-yne de formule:

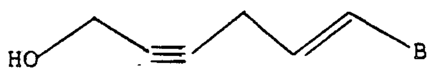

caractérisé en ce B est choisis parmi H, un groupe phényle, un groupe phényle substitué, un groupe alcoyle et un groupe $(CH_2)_nCOOH$ (où n = 0—5).

9. Procédé selon la revendication 1, dans lequel le produit final est un composé pent-4-ène-1-yne de formule:

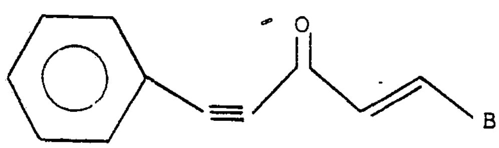

caractérisé en ce B est choisis parmi un groupe phényle substitué, un groupe $(CH_2)_nCOOH$ (où n = 0—5) et un groupe furyle.

10. Procédé selon la revendication 1, dans lequel le produit final est un composé pent-4-ène-1-yne de formule:

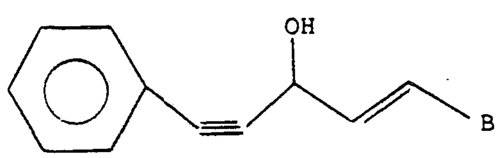

caractérisé en ce B est choisis parmi un groupe, phényle substitué, un groupe alcoyle, un groupe $(CH_2)_nCOOH$ (où n = 0—5) et un groupe furyle.